(19) Europäisches Patentamt
European Patent Office
Office européen des brevets

(11) Publication number: 0 155 165
A2

(12) # EUROPEAN PATENT APPLICATION

(21) Application number: 85301657.4

(22) Date of filing: 11.03.85

(51) Int. Cl.[4]: C 07 F 9/24, C 07 K 1/00

(30) Priority: 13.03.84 JP 47380/84

(43) Date of publication of application: 18.09.85
Bulletin 85/38

(84) Designated Contracting States: CH DE FR GB IT LI

(71) Applicant: AJINOMOTO CO., INC., 5-8,
Kyobashi 1-chome, Chuo-ku, Tokyo 104 (JP)

(72) Inventor: Hijiya, Toyoto, No. 2-210-1, Negishi-cho,
Yokosuka-shi Kanagawa-ken (JP)
Inventor: Nomura, Masayuki, No. 2297-2, Okazu-cho
Totsuka-ku, Yokohama-shi Kanagawa-ken (JP)
Inventor: Saito, Hideomi, No. 143, Aihara,
Sagamihara-shi Kanagawa-ken (JP)
Inventor: Ozawa, Yoichi, No. 2668-10, Kamariya-cho
Kanazawa-ku, Yokohama-shi Kanagawa-ken (JP)

(74) Representative: Bond, Bentley George et al, Haseltine
Lake & Co. 28 Southampton Buildings Chancery Lane,
London WC2A 1AT (GB)

(54) **Process for introducing phosphinoyl groups into amino groups.**

(57) A phosphinoyl group is introduced into an amino group of an amino acid or peptide to produce an N-(phosphinoyl) derivative of the amino acid or peptide, by a process which comprises subjecting the amino acid or peptide to a condensation reaction with a phosphorous acid diester having the general formula:

$$(RO)_2\overset{\displaystyle O}{\overset{\|}{P}}H$$

wherein R is an alkyl, aryl or aralkyl group, in a solvent comprising water and carbon tetrachloride and consisting of two phases, and in the presence of an alkali. The process is also applicable to esters of amino acids and peptides.

EP 0 155 165 A2

PROCESS FOR INTRODUCING PHOSPHINOYL GROUPS INTO AMINO GROUPS

This invention relates to a process for introducing phosphinoyl groups into amino groups of amino acids or peptides, or esters of amino acids or peptides, to produce N-(phosphinoyl) derivatives of the amino acid or peptides. These N-(phosphinoyl) derivatives are expected to be useful as drugs, for example as anti-hypertensive drugs (see JP-A-58126896, JP-A-5538382 and EP-A-0085488).

The introduction of a dibenzyloxyphosphinoyl group into an amino group of an amino acid or peptide can be performed by utilizing the so-called Schotten-Baumann reaction, in which debenzyloxyphosphinoyl chloride (hereinafter abbreviated to DBPCl) is added to an aqueous solution of amino acid or peptide in the presence of an alkali, resulting in a condensation reaction with the elimination of HCl.

With regard to the production of DBPCl, there are known the method wherein dibenzylphosphite (hereinafter abbreviated to DBPH) is treated with chlorine (J. Chem. Soc., 382 (1945)), and the method wherein DBPH is treated with $SO_2Cl_2$ (J. Chem. Soc., 1106 (1948)). However, these methods are defective in that poisonous gases such as $Cl_2$ and $SO_2$ are formed as by-products. Conversely, DBPCl can be produced by treating a dialkylphosphite with a tertiary amine such as triethylamine in carbon tetrachloride (J. Org. Chem, 15,

637 (1950)). In this method, no poisonous gas is produced. However, this method is defective in that DBPCl decomposes during its production or storage, since it is a highly reactive substance. Therefore, more than one mol of DBPCl is needed per mol of amino acid or peptide. Also, the decomposition product may contaminate the desired N-(dibenzyloxyphosphinoyl) amino acid or N-(dibenzyloxyphosphinoyl) peptide.

There are known methods which do not isolate DBPCl as an intermediate product. Thus, it is known to add di-para-substituted benzylphosphite in $CCl_4$ to cyclohexylamine or aqueous ammonia to introduce a N-di-para-substituted benzyloxyphosphinoyl group, thereby producing di-para-substituted benzylcyclohexylaminophosphonate or di-para substituted benzylaminophosphonate (J. Am. Chem. Soc., 77, 3522 (1955)). However, in these methods, the HCl produced reacts with the unreacted amino compound to form the amine hydrochloride which does not participate in the reaction. Therefore, more than two equivalents of amino compound are needed per equivalent of di-para-substituted benzylphosphite. As an example of a method in which the introduction of a N-dibenzyloxyphosphinoyl group is performed by using equivalent amounts DBPH and amino compound, it is known (Synthesis, 1975, 509) that a phase transfer catalyst such as benzyltriethylammonium chloride can be used; however, the amino used is soluble in $CCl_4$, which is considered as a substitute for the tertiary amines such as trimethylamine given in the above-mentioned document (see J. Org. Chem., 15, 637 (1950)).

It is the object of the present invention to provide a novel and a simple process for producing an N-(phosphinoyl)derivative of an amino acid or peptide, or of an ester of an amino acid or peptide, by introducing a phosphinoyl group into an amino group of the amino acid or peptide or ester thereof,

which is not soluble in carbon tetrachloride but soluble in water, with high yield.

We have found, after detailed investigation, that the introduction of a phosphinoyl group into an amino group proceeds when phosphites (namely phosphorous acid diesters) are made to react with an amino acid or peptide, or an ester thereof, in a solvent containing water and carbon tetrachloride and consisting of two phases, in the presence of an alkali.

Thus, the present invention relates to a process for introducing a phosphinoyl group into an amino group of an amino acid or petide or an ester thereof, characterized in that the amino acid or peptide, or the ester thereof, and a phosphorous acid diester (such as a dialkylphosphite, a diarylphosphite or a diaralkylphosphite) are subjected to a condensation reaction in one step in a solvent containing water and carbon tetrachloride and consisting of two phases, in the presence of an alkali.

The phosphorous acid diester has the formula:

$$(RO)_2\overset{O}{\overset{\|}{P}}H$$

wherein R is a hydrocarbon group, preferably an alkyl, aryl or aralkyl group.

As the solvent, any solvent containing water and carbon tetrachloride and consisting of two phases can be used. Other components can also be present in the solvent. Examples of the alkali are NaOH, KOH, $Na_2CO_3$ and $NaHCO_3$.

As the amino acid or peptide, or ester thereof, used in this invention, those which are insoluble in carbon tetrachloride but soluble in water can be used. When the amino acid or peptide, or the ester thereof, has functional groups besides the amino group into which the phosphinoyl group is to be introduced, all or some of the functional groups can be

protected with a protective group commonly used; however, the amino acid or peptide, or the ester thereof, should be insoluble in carbon tetrachloride and soluble in water.

Examples of amino acids which can be used in this invention are neutral amino acids such as alanine and proline, acidic amino acids such as glutamic acid and aspartic acid, and basic amino acids such as lysine and ornithine.

Examples of peptides which can be used in this invention are alanyl proline, alanyl prolyl proline, α-aspartyl-phenylalanine, glutamyl-prolyl-proline, lysyl-prolyl-proline, methionyl-prolyl-proline, and alginyl-prolyl-proline, and examples of peptide esters which can be used in this invention are the foregoing peptides in which the carboxylic group is esterified by a lower alkyl group.

Examples of the reagents for introducing the phosphinoyl group are dialkylphosphites whose alkyl groups contain from 1 to 12 carbon atoms (such as dimethyl phosphite and diethyl phosphite); diaryl phosphites whose aryl groups contain from 6 to 11 carbon atoms (such as diphenyl phosphite and di(alkyl-substituted phenyl) phosphite); and diaralkyl phosphites whose aralkyl groups contain from 7 to 12 carbon atoms (such as dibenzyl phosphite and di(alkyl-substituted benzyl) phosphite).

Since the reaction is performed in a two-phase system of water and $CCl_4$, according to this invention, the yield can be raised by improving the agitation efficiency. The yield can also be improved by using a medium which is soluble in both the water and $CCl_4$ used as the solvent. Examples of such media are ethers (such as dioxane and tetrahydrofuran), ketones (such as acetone), and lower alcohols (such as methanol and ethanol). When those media are used, there is no limit upon the amounts used, but it is preferable

to use from 1 to 4 volumes thereof for 1 volume of the alkaline solution of amino acid or peptide or ester thereof. These media are usually added to the aqueous solution of amino acid or peptide,or ester thereof, before the addition of the phosphite (such as DBPH), but can also be added to the solution of the phosphite (such as DBPH) in $CCl_4$.

The reaction system is preferably kept alkaline, at pH of more than 8. However, when a peptide is used, the pH is preferably kept above 10, and, if the pH is maintained above 13, the production of by-products can be restrained. When a peptide ester is used, a pH of from 8 to 10 is preferable, in order to prevent saponification of the ester. The concentration of the aqueous solution of amino acid or peptide, or ester thereof, does not affect the reaction yield, but the concentration generally used is from 0.1 to 2 mol/litre. The molar ratio of phosphite (such as DBPH) to amino acid or petide, or ester thereof, is preferably from 0.8:1 to 2:1, more preferably from 1.0:1 to 1.3:1.

The molaration of $CCl_4$ to the phosphite (such as DBPH) is preferably more than 1:1, more preferably from 1:1 to 13:1.

When the time over which the phosphite such as DBPH is added to the $CCl_4$ solution is reduced, side reactions occur easier, but no problem occurs when the phosphite is added over a period of more than 30 minutes. It is usually added over a period of 1 to 2 hours. The reaction is usually completed within 30 minutes to 1 hour after the addition of the phosphite such as DBPH. When the temperature of the reaction system is high, side reactions occur easier; therefore the reaction is usually performed at a temperature of from 0°C to 40°C. During the reaction, HCl is produced and accordingly the pH of the reaction liquid becomes lower; therefore,

a base is used as mentioned above in order to maintain the pH value within the alkaline range.

By acidifying the aqueous phase of the reaction liquid thus obtained, the required product, for example an N-(dibenzyloxyphosphinoyl) derivative of an amino acid or peptide, or ester thereof, can be produced. When a water soluble peptide ester, such as L-alanyl-L-prolyl-L-proline methyl ester, is used as raw material, N(dibenzyloxyphosphinoyl)-L-alanyl-L-prolyl-L-proline (DBPAPP) can be produced by, for example, the following procedure. By adding strong base such as NaOH, to the solution in which the reaction was performed within the pH range of 8 to 10, the ester is saponified, and then after separating the $CCl_4$ phase, the aqueous phase is acidified and thus DBPAPP is obtained.

As is evident from the above description, DBPH, for instance, and an amino acid or peptide, or an ester of an amino acid or peptide, can be subjected to a condensation reaction in one step without isolating the highly reactive intermediate such as DBPCl, and therefore this invention is very useful industrially.

The present invention will now be illustrated by the following Examples.

EXAMPLE 1

5.66g (20 mmol) of L-alanyl-L-prolyl-L-proline was dissolved in 12.2 ml of water, 15.0 ml of acetone were added to this solution, the pH of the solution was adjusted to 14.0 with 2N aqueous NaOH solution, and the solution was vigorously stirred while being cooled with ice. To this solution, 5.40g (20.6 mmol) of dibenzylphosphite (DBPH) in $CCl_4$ (5 ml) were added over a period of 1 hour, and then the solution was agitated for 2 hours. The pH of the aqueous solution was maintained in the range of 13.0 to 14.0 during the above procedure. After separating the carbon tetrachloride phase and the aqueous phase, the aqueous phase was washed with 30 ml of ethylene dichloride.

To the aqueous phase thus obtained, 6N HCl was

added under agitation with ice cooling until the liquid became turbid whitish. The pH was 4.05 at this time. Then 6N HCl was added to the solution intermittently and the solution was agitated for 6 hours under ice cooling while the pH was kept around 4.0. To the slurry produced, 6N HCl was added and the pH was adjusted to 3.0, and then the slurry was agitated for another 2 hours. From this slurry, crystals of N-(dibenzyloxyphosphinoyl)-L-alanyl-prolyl-L-proline (DBPAPP) were separated by filtration, washed with 10 ml of water and dried.

Dried Weight: 9.54 g

Purity: 98.0% (High Pressure Liquid Chromatography (HPLC))

Yield based on L-alanyl-L-prolyl-l-proline: 86.0%

Elementary Analysis as DBPAPP:

Calculated Values: H6.30%, C59.66%, N7.73%

Analyzed Values: H6.27%, C59.63%, N7.71%

EXAMPLE 2

5.66g (20 mmol) of L-alanyl-L-prolyl-L-proline were dissolved in 12.2 ml of water, 38.6 ml of dioxane were then added to this solution, and the pH of the solution was then adjusted to 9.6 with 10% aqueous $Na_2CO_3$ solution. To this solution, 8.39g (32 mmol) of DBPH in $CCl_4$ (10 ml) were added over a period of 2 minutes, while the mixture was agitated vigorously, at room temperature. Then the solution was agitated for 4 hours while the pH was kept around 10 . After the aqueous phase had been separated from the carbon tetrachloride phase, the DBPAPP contained in the aqueous phase was deterimed by HPLC.

Yield: 82.8% based on L-alanyl-L-prolyl-L-proline)

EXAMPLE 3

3.72g (20 mmol) of L-alanyl-L-proline were dissolved in 13ml of water, and 40ml of acetone were added to this solution. The pH of the solution was adjusted to 13.0 with 2N aqueous NaOH solution. To this

solution, 6.56g (25 mmol of DBPH in $CCl_4$(10 ml) were added over a period of 1 hour, and then the solution was agitated for 1 hour. After the aqueous phase had been separated from the carbon tetrachloride phase, the N-(dibenzzyloxyphosphinoyl)-L-alanyl-L-proline in the aqueous phase was determined by HPLC.

Yield: 57.8% (based on L-alanyl-L-proline)

EXAMPLE 4

9.38g (20 mmol) of L-alanyl-L-prolyl-L-proline methyl ester p-toluenesulphonate were dissolved in 20 ml of water, and then 40 ml of dioxane were added. To this solution, 8.39 g (32 mmol) of DBPH in $CCl_4$ (10 ml) were added over 35 minutes while the pH was adjusted to 8.0 with 10% aqueous $Na_2CO_3$ solution, and then the solution was agitated for 3 hours while the pH was held in the range of 7.5 to 9.0. To this reaction liquid, 25% aqueous NaOH solution was added so that the pH was approximately 13, and then the solution was agitated for 2 hours at room temperature. After the aqueous phase had been separated from the carbon tetrachloride phase, the DBPAPP in the aqueous phase was determined by HPLC.

Yield: 93.3% (based on L-alanyl-L-prolyl-L-proline methyl ester p-toluenesulphonate)

EXAMPLE 5

5.66 g (20 mmol) of L-alanyl-L-prolyl-L-proline were dissolved in 20ml of water, and the pH of the solution was adjusted to 11.7 with 2N NaOH. To this solution, 5.7 g (21.7 mmol) of DBPH in $CCl_4$ (4.2 ml) were added over a period of 1 hour while vigorously agitating the mixture and cooling it with ice. The agitation was continued for another 1.5 hours while the pH was held around 11. After the aqueous phase had been separated from the carbon tetrachloride phase, the DBPAPP in the aqueous phase was determined by HPLC.

Yield: 50.7% (based on L-alanyl-L-prolyl-L-proline)

EXAMPLE 6

5.63g (20.1 mmol) of α-L-aspartyl-L-phenylalanine were dissolved in 15 ml of water, and after 15 ml of acetone had been added, the pH was adjusted to 12.0 with 2N NaOH. To this solution, 5.6g (21.4 mmol) of DBPH in $CCl_4$ (15 ml) were added over a period of 1 hour with vigorous agitation and ice cooling, and the agitation was continued for another 1 hour while the pH was held around 12. After the aqueous phase had been separated from the carbon tetrachloride phase, the aqueous phase was washed twice with 30 ml of ethylene dichloride. To this aqueous phase, 60 ml of ethylene dichloride were added, the pH was adjusted to 2.5 with 6N HCl, the solution was agitated, and then the required product was extracted . Thus, the ethylene dichloride phase was separated and washed with 40 ml of water, and then it was concentrated under reduced pressure until it became oily. Hexane was then added to the residue obtained and crystals formed. The crystals were separated by filtration, and recrystallized from methanol/ethyl acetate/hexane.

Weight of the crystals: 6.86 g

Yield: 63.1% (based on α-L-aspartyl-L phenylalanine)

EXAMPLE 7

1.69 g (14.70 mmol) of L-proline was dissolved in 8.8 ml of water, and 22 ml of acetone were added to this solution. The pH of the solution was adjusted to 14.2 with 5N aqueous NaOH solution, and then the solution was vigorously agitated under ice cooling. 4.63 g (17.67 mmol) of DBPH in $CCl_4$ (8.8 ml) were added to the solution over 30 minutes under agitation, and the agitation was continued for another 30 minutes, while the pH of the aqueous solution was kept in the range of 13.2 to 14.4. 10 ml of water and 20 ml of ethylene dichloride were added to the reaction liquid. The aqueous phase was separated from the

ethylene dichloride phase, and the aqueous phase was washed with 20 ml of ethylene dichloride. 40 ml of ethylene dichloride were added to the aqueous phase obtained, then 1 N HCl was added until the pH reached 3.0, and then the ethylene dichloride phase was separated from the aqueous phase. After washing the ethylene dichloride phase obtained, with 20 ml of water, the ethylene dichloride was removed by distillation under reduced pressure. 2.66 g (14.70 mmol) of dicyclohexylamine were added to the oily residue, then hexane was added, and crystals precipitated. The resulting N-(dibenzyloxyphosphinoyl)-L-proline dicylcohexylamine salt obtained by filtration was washed with 10 ml of hexane, and dried.

Weight of the crystals: 7.07g (12.72 mmol)
Yield based on L-proline: 86.5%
Elementary analysis:
Calculated values: H 8.15%, C 66.89%, N 5.03%
Analyzed values: H 8.11%, C 67.00%, N 5.16%

EXAMPLE 8

2.50 g (21.74 mmol) of L-proline were dissolved in 13 ml of water, 32 ml of acetone were added, the pH of the solution was adjusted to 11.0 with 2N aqueous NaOH solution, and then the solution was agitated vigorously under cooling with ice. To this solution, 6.11g (26.09 mmol) of phosphorous acid diphenyl ester in $CCl_4$ (13 ml) were added over a period of 40 minutes with agitation, and the agitation was continued for another 1 hour, while the pH of the aqueous solution was held in the range of 10.7 to 11.2. 35 ml of water and 30 ml of ethylene dichloride were added to the reaction liquid, the aqueous phase was separated from the ethylene dichloride phase, and then the aqueous phase was washed with 30 ml of ethylene dichloride. 60 ml of ethylene dichloride were added to the aqueous phase obtained, and then 6N HCl was added to the solution until the

pH became 2.8. The ethylene dichloride phase was then separated from the aqueous phase. The ethylene dichloride phase obtained was washed with 20 ml of water, then concentrated under reduced pressure and the crystals separated.

Weight of the crystals: 5.45 g (15.72 mmol)

Yield based on L-proline: 72.3%

Elementary analysis:

Calculated values: H 5.22%, C 58.79%, N 4.03%

Analyzed values: H 5.36%, C 58.60%, N 4.06%

EXAMPLE 9

5.00 g (43.48 mmol) of L-proline were dissolved in 26 ml of water, and then 65 ml of acetone were added. The pH of the solution was adjusted to 11.5 with 1N aqueous NaOH aqueous solution, and the solution was agitated vigorously under ice cooling. To this solution, 7.21g (52.25 mmol) of phosphorous acid diethyl ester in $CCl_4$ (26 ml) were added over a period of 50 minutes under agitation, and the solution was then agitated for another 40 minutes, while the pH of the aqueous solution was kept in the range of 11.2 to 11.6. 30 ml of water and 60 ml of ethylene dichloride were added to the reaction solution, the aqueous phase was separated from the ethylene dichloride phase, and the aqueous phase was washed with 50 ml of ethylene dichloride. 120 ml of ethylene dichloride weere added to the aqueous phase obtained, 6N HCl was added until the pH became 2.9, and then the ethylene dichloride phase was separated from the aqueous phase. The ethylene dichloride phase obtained was washed with 60 ml of water, and the ethylene dichloride was removed by distillation under reduced pressure. Ethyl acetate was added to the oily residue, and then 4.72 g (26 mmol) of dicyclohexylamine were added, and crystals was precipitated. The resulting N-(diethoxyphosphinoyl)-L-proline dicyclohexylamine salt obtained by filtration was washed with ethyl acetate

and then dried.

Weight of the crystals: 10.99 g (25.31 mmol)

Yield based on L-proline: 58.2%

Elementary analysis:

Calculated values: H 9.55%, C 58.32%, N 6.48%

Analyzed values: H 9.37%, C 58.36%, N 6.41%

CLAIMS

1. A process for introducing a phosphinoyl group into an amino group of an amino acid or peptide, or of an ester of an amino acid or peptide, to produce an N-(phosphinoyl) derivative thereof, which process comprises subjecting an amino acid or peptide, or an ester of an amino acid or peptide, to a condensation reaction with a phosphorous acid diester having the general formula:

$$(RO)_2\overset{O}{\overset{\|}{P}}H$$

wherein R is an alkyl, aryl or aralkyl group, in a solvent comprising water and carbon tetrachloride and consisting of two phases, and in the presence of an alkali.

2. A process acccording to claim 1, wherein the solvent additionally contains a medium which is soluble in both water and carbon tetrachloride.

3. A process according to claim 2, wherein the medium is an ether, a ketone or a lower alcohol.

4. A process according to any of claims 1 to 3, wherein the amino acid or peptide, or the ester of an amino acid or peptide, contains one or more functional groups other than the amino group into which the phosphinoyl group is to be introduced, and wherein one or more of these functional groups is protected.

5. A process according to any of claims 1 to 4, wherein the reaction system has a pH of more than 8.

6. A process according to any of claims 1 to 5, wherein the reaction is carried out at a temperature of from 0 to 40°C.

7. A process according to any of claims 1 to 6, wherein the molar ratio of the diester to the amino acid or peptide, or to the ester of an amino acid or peptide, is from 0.8:1 to 2:1.

8. A process according to any of claims 1 to 7, wherein the molar ratio of carbon tetrachloride to the amino acid or peptide, or to the ester of an amino acid or peptide, is from 1:1 to 13:1.

9. A process according to any of claims 1 to 8, wherein the diester is a dialkylphosphite whose alkyl groups contain from 1 to 12 carbon atoms, a diarylphosphite whose aryl groups contain from 6 to 11 carbon atoms, or a diaralkyl phosphite whose aralkyl groups contain from 7 to 12 carbon atoms.

10. A process according to any of claims 1 to 9, wherein the alkali is NaOH, KOH, $Na_2CO_3$ or $NaHCO_3$.